# EUROPEAN PATENT SPECIFICATION

(11) **EP 3 116 548 B1**
(45) Date of publication and mention of the grant of the patent: **17.02.2021**
(21) Application number: 15715798.3
(22) Date of filing: 11.03.2015
(51) Int. Cl.: A61K 47/26, A61K 9/08, A61K 31/35, A61K 33/26

(54) **COMPOSITION**
ZUSAMMENSETZUNG
COMPOSITION

(30) Priority: 12.03.2014 GB 201404390
(43) Date of publication of application: 18.01.2017
(73) Proprietor: Shield TX (UK) Limited, Gateshead Quays NE8 3DF (GB)
(72) Inventor: STOCKHAM, Michael Arthur, Saffron Walden Essex CB11 4QX (GB)
(74) Representative: Potter Clarkson
(86) International application number: PCT/GB2015/050711
(87) International publication number: WO 2015/136282

(56) References cited:
- EP-A2- 0 159 917
- WO-A1-03/097627
- WO-A1-2012/101442
- AHMET M.T. ET AL.: "A potential iron pharmaceutical composition for the treatment of iron-deficiency anaemia. The crystal and molecular structure of mer-tris-(3-hydroxy-2-methyl-4H-pyran-4-on ato)iron(III)", JOURNAL OF THE CHEMICAL SOCIETY, DALTON TRANSACTIONS, CHEMICAL SOCIETY. LETCHWORTH, GB, 1 January 1988 (1988-01-01), pages 1159-1163, XP008150796, ISSN: 1472-7773

## Description

The invention relates to compositions in the form of a liquid or a liquid suspension comprising an iron hydroxypyrone.

The hydroxypyrones maltol and ethyl maltol are known to improve the flavor and aroma of foods and this is disclosed in US 3,376,317.

Iron complexes have also been used to impart a red colour to foods and this is described in US 4,018,907 and US 4,018,934.

US 2004/0029853 A1 discloses that solid forms comprising a mixture of a ferrous salt and a hydroxypyrone can be used to increase the level of iron in a patient's bloodstream. The pharmaceutical composition can also be a suspension in liquid form.

US 2005/0250754 A1 describes a method that produces ferric trimaltol.

US 8,080,520 B2 discloses the use of alditols such as mannitol, maltitol and xylitol as suitable excipients for the iron product, heme iron, which is used in combination with a second iron source. The product is iron reacted with the peptide and is not haem iron in a porphyrin ring. The patent cites many iron compounds.

CN 1078395A describes an oral liquor composed of composite amino-acid, Fe²⁺, Zn²⁺, Vit B1, Vit B2, Vit C, fruit of Chinese wolfberry, longan, haw, malt, jujube, Chinese yam, and crystal sugar.

JPH 0367571A discloses an iron component-enriched soft drink obtained by incorporating maltol and/or ethyl maltol in an iron component-enriched soft drink that contains an iron compound.

US 2,822,317 describes a liquid iron-ascorbic acid preparation which is said to be useful as a hematinic.

Liquid preparations such as ferrous sulphate elixir (USP 1995) contain sucrose and a ferrous gluconate mixture (Martindale 1989) contains glucose. These ferrous iron products have a "metallic" sour/bitter taste.

There is a clear distinction between pharmaceutical forms as a capsule, where the powders are added in a dry form, and as prospective liquid form where taste, which is a function detected on areas of the tongue, affects acceptability. Taste is far less important when a capsule or a tablet is swallowed whole or when a tablet is coated. As a result the known liquid ferrous iron products have included very high amounts of glucose or sucrose as a sweetening agent.

Certain patients may not be able to take iron products in capsule or tablet form. This particularly applies to children and old people. Existing liquid preparations of ferrous sulphate or ferric ammonium citrate, for example, have many disadvantages and are therefore not extensively used. Furthermore, administering medicines to children requires the most palatable formulation.

There is a need for a stable, acceptable liquid formulation of iron hydroxypyrones.

The present invention recognises that iron hydroxypyrones, particularly ferric hydroxypyrones, possess a caramelic metallic taste but only at higher molar concentrations. Following taste studies carried out with these iron hydroxypyrones it was found that they have a metallic taste at higher concentrations, although this is significantly less than ferrous salts. This led to the recognition that in a liquid formulation with, for example, an iron hydroxypyrone particularly at medicinal levels, such as 1 to 150 mg or 30-120 mg per dose (as iron), there is a need for taste masking.

There is another advantage of a liquid formulation of a ferric tri (hydroxypyrone) in that there is no need to have an acidified preparation for either efficacy or stability. In fact, as ferric trimaltol disproportionates at acid pH values there is a distinct advantage for the liquid formulation to have a near neutral pH to confer stability. This is a significant difference between ferric pyrones in solution and ferrous salts used in medicine.

In a first aspect, the present invention provides a composition in the form of a liquid, liquid suspension or semisolid, comprising an iron hydroxypyrone and a taste masking agent, and wherein the iron hydroxypyrone is preferably present in the liquid, liquid suspension or semisolid in a molar concentration of at least about 10⁻⁵ M (mol/L).

In a second aspect there is provided a composition according to the invention, for use in medicine.

In a third aspect, there is provided a composition according to the invention, for use in treating and/or preventing anaemia, or for increasing the level of iron in a subject's body, such as a subject's bloodstream. The anaemia is preferably iron deficiency anaemia.

In a further aspect, there is provided a method for treating and/or preventing anaemia in a patient, or for increasing the level of iron in a subject's body, for example bloodstream, comprising the step of administering to the patient or subject a composition according to the invention.

In another aspect, there is provided a method of forming a liquid, liquid suspension or semisolid, such as according to the invention, which comprises combining an iron hydroxypyrone, such as in a solid or dry form, with a liquid and a taste masking agent.

In yet another aspect, there is provided a kit of parts comprising: an iron hydroxypyrone in the form of a solid; one or more liquids; and one or more taste masking agents, preferably wherein the amount of the iron hydroxypyrone and the one or more liquids is such that, when combined, a liquid or liquid suspension is formed in which the iron hydroxypyrone is present in a molar concentration of at least about 10⁻⁵ M.

For example, the volume of the liquid in the kit may be from about 5 ml to about 1500 ml, such as from about 10 ml to about 500 ml and the amount of the iron hydroxypyrone in the kit may be from about 5 mg to about 100 g, such as from about 100 mg to about 10 g.

The iron hydroxypyrone, one or more liquids, and one or more taste masking agents may be as defined in any of the embodiments herein. The molar concentration of the iron hydroxypyrone may be as defined in any of the embodiments herein.

In one embodiment of the kit of parts, the one or more liquids may comprise the one or more taste masking agents or the iron hydroxypyrone component may comprise the one or more taste masking agents. For example, the kit of parts may comprise a liquid dispenser for the one or more liquids and any components therein and a container for the solid components. A suitable container for the solid components could be, for example, a sachet. The solid components may be in the form of a dry or dried powder, such as defined herein.

In one embodiment, the kit of parts may optionally comprise any of the further components specified herein in the defined amounts.

The iron hydroxypyrone, taste masking agent and liquid may be as defined in any of the embodiments disclosed herein. The amounts of these components may also be as defined in any of the embodiments disclosed herein.

Preferably, the composition of the invention as defined in any of the embodiments herein does not comprise an amino-acid complex and/or sugar, such as crystal sugar or isomerized liquid sugar, for example isomerized liquid sugar having a solid content of about 75%. Additionally, or alternatively, preferably the composition of the invention as defined in any of the embodiments herein does not comprise ascorbic acid, for example, ascorbic acid in the free acid form or as a derivative thereof, such as a salt or ester of ascorbic acid.

Typically, the composition of the invention is substantially free, or free of, vitamins, such as, for example, vitamins selected from Vitamin C, Vitamin B1, or Vitamin B2 and combinations thereof.

Preferably, the composition of the invention is not an oral care composition, such as, for example, a toothpaste. For example, the composition of the invention, as defined in any of the embodiments herein, is preferably a beverage or a foodstuff. When the composition of the invention is in the form of a beverage, that beverage may be a non-soft drink.

The composition of the invention is in the form of a liquid or a liquid suspension. Thus, the composition of the invention is not in the form of a solid, such as a powder, tablet or capsule. The term "liquid" is intended to include a solution of the iron hydroxypyrone in a solvent, such as water. In a liquid form, the iron hydroxypyrone is generally dissolved in the solvent to form a continuous phase. The term "liquid" can also encompass emulsions such as oil-in-water liquid emulsions, or water-in-oil liquid emulsions but in one embodiment of the invention, emulsions are not encompassed. The term "liquid" may or may not also encompass a semisolid. In an embodiment of the invention, the composition as defined in any of the embodiments herein is in the form of a semisolid. A semisolid typically does not hold its shape like a solid but does not flow like a liquid. Examples of semisolids include, for example, foodstuffs such as yoghurt or mayonnaise.

The liquid or liquid suspension of the invention may also be fully or partially frozen subsequent to its formation. In one embodiment, the liquid or liquid suspension is not fully or partially frozen after formation.

The term "liquid suspension" is intended to mean a composition which comprises a suspension of the iron hydroxypyrone in a liquid medium, for example an aqueous or non-aqueous liquid. The suspension may also comprise dissolved iron hydroxypyrone. In the case where the solubility of the iron hydroxypyrone is exceeded, the composition may comprise a mixture of dissolved and non-dissolved iron hydroxypyrone. In a suspension, the iron hydroxypyrone is generally suspended in the liquid medium. The iron hydroxypyrone, such as ferric trimaltol, may be visible as suspended particles.

In one embodiment of the invention, the liquid comprises an aqueous solution. The aqueous solution comprises or preferably consists of water. A non-aqueous liquid may include, for example, oils and/or alcohols, or other pharmaceutically acceptable liquids.

The term "iron hydroxypyrone" as used herein is intended to include compositions which comprise a hydroxypyrone and iron. The term includes, for example, complexes of iron with a hydroxypyrone, such as, for example, ferric trimaltol, as well as mixtures comprising an iron compound, such as a salt or a complex of iron, and a hydroxypyrone, preferably in a substantially non-complexed form (such as less than 10%, 5%, 2%, or 1% hydroxypyrone complexed), for example ferrous gluconate and maltol or ferric maltol gluconate, preferably in the solid state.

In an embodiment of the invention, the term "iron hydroxypyrone" refers to a 1:3 molar complex of ferric iron to hydroxypyrone. This complex is neutral. The term "iron hydroxypyrone" may or may not include carboxylic acids as counterions. In addition, the term may or may not include charged complexes of iron with hydroxypyrone, such as 1:1, or 1:2 molar complexes of iron to hydroxypyrone, with counteranions, such as carboxylate anions. In one embodiment of the invention, the molar ratio of iron to hydroxypyrone is 1: at least 3.

In one embodiment of the invention, the iron hydroxypyrone has an undesirable taste, particularly at any of the concentrations specified herein. The undesirable taste may be, for example, a sour and/or bitter and/or metallic taste.

In an embodiment of the invention, the term "iron hydroxypyrone" refers to mixture comprising or consisting of a ferrous or ferric salt and a hydroxypyrone. In one embodiment of the invention the mixture does not comprise a diluent or a water soluble inert bulking agent.

In an embodiment of the invention, the iron, or combination of iron and hydroxypyrone, or ferric iron, or combination of ferric iron and hydroxypyrone, is not co-dried on an edible inert diluent or bulking agent.

The ferric salt can be an iron (III) salt with any pharmaceutically acceptable anion. In one embodiment, the ferric salt can be an iron (III) inorganic salt, such as ferric sulphate or a ferric halide, such as ferric chloride. Alternatively, the ferric salt can be an iron (III) organic salt, such as, for example, a ferric carboxylate such as ferric citrate. In one embodiment of the invention, the iron (III) salt does not comprise ferric nitrate.

The ferrous salt can be an iron (II) salt with any pharmaceutically acceptable anion. Preferably, the iron (II) salt is iron (II) carbonate or an iron (II) carboxylate. Suitable iron (II) carboxylates include, for example, iron (II) gluconate, iron (II) succinate, and iron (II) fumarate. These ferrous salts are readily available at pharmaceutically acceptable levels of purity.

The molar ratio of the ferric or ferrous salt to the hydroxypyrone is preferably from 1:1 to 1:10, such as about 1:5, 1:4.4, 1:4 or 1:3.

In one embodiment, the iron hydroxypyrone is preferably present in the liquid or liquid suspension in a molar concentration of at least about 10⁻⁵ M (mol/L). For example, the iron hydroxypyrone may be present in a molar concentration of from about 10⁻⁵ M to about 1 M, such as from about 10⁻⁴ M to about 10⁻¹ M, or from about 10⁻³ M to about 10⁻² M, such as from about 10⁻² M to about 10⁻¹ M. Typically, the iron hydroxypyrone is present in a molar concentration of at least about 10⁻⁴ M, or at least about 10⁻³ M.

In one embodiment of the invention, the iron hydroxypyrone is present in the liquid or liquid suspension in a concentration of from about 0.05 to about 1 M, such as from about 0.1 to about 0.8 M or from about 0.2 to about 0.6 M, or from about 0.3 to about 0.5 M, or from about 0.1 to about 0.5 M. Preferably, the iron hydroxypyrone is present in the liquid or liquid suspension in a concentration of at least about 0.04M, preferably at least about 0.05 M, such as greater than or equal to about 0.08 M. When the concentration is at least about 0.04 M, optionally the composition does not comprise Vitamin C.

In an embodiment of the invention, the composition of the invention, as defined in any of the embodiments herein, is provided in any of the above defined concentrations and subsequently diluted before use with the liquid, such as water. The dilution factor (molar concentration after dilution/molar concentration before dilution) may be for example less than about 0.1, 0.2, 0.5, or 0.01.

The iron hydroxypyrone is typically the sole or only source of iron in the composition but other iron sources may be used in certain embodiments. In one embodiment of the invention, the iron hydroxypyrone is the sole source of iron in the composition. In another embodiment of the invention the composition does not comprise heme iron and/or heme iron polypeptide. The iron hydroxypyrone may be produced according to the methods disclosed in, for example, WO 03/097627 and WO 2012/101442, the disclosure of which is incorporated herein by reference.

The molar concentration is preferably based on the molar amount of the total components of the iron hydroxypyrone added to a solvent or liquid medium. For example, if the iron hydroxypyrone comprises a 1:3 molar complex of iron to hydroxypyrone, this complex can form the basis of the molar concentration calculation and if there is additional uncomplexed hydroxypyrone this may, optionally, be used in the calculation. On the other hand, if the iron hydroxypyrone comprises a mixture of a ferric or ferrous salt and a hydroxypyrone, the total amount of these components may form the basis for the molar concentration calculation.

The taste masking agent may be any agent which is capable of preventing, modifying, reducing or avoiding any undesirable taste which is associated with the iron hydroxypyrone and which would otherwise exist in the absence of the masking agent. For example, the taste masking agent may prevent, modify and/or reduce any undesirable sour and/or bitter and/or metallic taste associated with the iron hydroxypyrone. As such, the taste masking agent is not used as an excipient or as a carrier or bulking agent. This can be reflected in, for example, smaller amounts of the taste masking agent in the composition compared to what would be typical levels for excipient or bulking agents. Thus, the amount of the taste masking agent may be less than about 90 % (w/v), less than 80% (w/v), less than 70 %

(w/v), less than 60 % (w/v) or less than about 50 % (w/v) based on the total volume of the composition.

In the invention, the taste masking agent is added in an amount effective to mask the taste of the iron hydroxypyrone at a particular concentration.

The taste masking agent, such as comprising an acyclic polyol, for example, a sugar alcohol or a non-saccharide sweetener, is typically present in the solution or suspension in a molar concentration of at least about 0.01 M, such as at least about 0.1 or 0.5 M. For example, the taste masking agent, such as a sugar alcohol, may be present in a molar concentration of from about 0.001 M to about 5 M, such as from about 0.01 M to about 3 or 4M, for example, from about 0.1 M to about 0.5 M or 1M. In an embodiment of the invention, the taste masking agent is present in the composition in an amount of from about 0.1 to about 70 % (w/v), or from about 1 to about 60 % (w/v), such as from about 5 to about 40 % (w/v).

The weight ratio of the iron hydroxypyrone to the taste masking agent in one embodiment of the invention is from about 1:1 to about 1:20, such as from about 1:2 to about 1:15, about 1:3 to 1:10, for example about 1:5.

In an embodiment of the invention, the taste masking agent comprises a sugar alcohol, stevia, a non-saccharide sweetener, such as aspartame, or combinations thereof

In one embodiment of the invention, the taste masking agent comprises one or more glycosides, such as a steviol glycoside. For example, the taste masking agent may comprise a *Stevia* extract, such as a steviol glycoside isolated from *Stevia* or may be the leaves or extract of a *Stevia* plant. The stevia or stevia-related taste masking or sweetening agent is preferably commercially available and typically comprises an extract from the leaves of the *Stevia* genus.

In an embodiment of the invention the taste masking agent comprises an acyclic polyol, or a non-sugar or non-saccharide sweetener.

Suitable examples of non-saccharide sweeteners include, for example, stevia, aspartame, sucralose, neotame, acesulfame potassium, or saccharin, and combinations thereof.

In one embodiment of the invention, the taste masking agent does not comprise a sugar. The term "sugar" is intended to include, for example, monosaccharides, disaccharides, trisaccharides, oligosaccharides and polysaccharides. For example, the taste masking agent does not typically comprise any one or more of glucose, lactose, fructose or sucrose. Preferably, the term "sugar" also includes crystal sugar and isomerized liquid sugar.

In an embodiment of the invention, the composition may further comprise a sugar, such as defined above. For example, in one embodiment of the invention, the composition may further comprise sucrose. The concentration of sugar, for example, sucrose, may be as defined above for the taste masking agent.

The amount of sugar, such as sucrose, can be reduced to less than about 20 % (w/v), for example, less than about 10 % (w/v), or less than about 5 % (w/v) based on the total volume of the composition in the presence of a non-saccharide sweetening agent, such as stevia.

In one embodiment of the invention, the taste masking agent comprises a sugar alcohol. A particular sugar alcohol may be obtained from a corresponding sugar using means known in the art. Typically, sugar alcohols are available commercially. These organic compounds are a class of polyols, also called polyhydric alcohol, polyalcohol, or glycitol. They are generally white, water-soluble solids.

Sugar alcohols, also known as polyols, occur naturally in foods and come from plant products such as fruits and berries. They provide fewer calories (about a half to one-third less calories) than regular sugar. This is because they are converted to glucose more slowly, require little or no insulin to be metabolized and do not cause sudden increases in blood sugar.

Common sugar alcohols are mannitol, sorbitol, xylitol, lactitol, isomalt, maltitol and hydrogenated starch hydrolysates (HSH).

Mannitol occurs naturally in pineapples, olives, asparagus, sweet potatoes and carrots. It is extracted from seaweed for use in food manufacturing. Mannitol has 50-70 percent of the relative sweetness of sugar.

Sorbitol is found naturally in fruits and vegetables. It is manufactured from corn syrup. Sorbitol has only 50 percent of the relative sweetness of sugar.

Xylitol is also called "wood sugar" and occurs naturally in for example straw, corncobs, fruit, vegetables, cereals and mushrooms. Xylitol has the same relative sweetness as sugar. Lactitol has about 30-40 percent of sugar's sweetening power, but its taste and solubility profile resembles sugar. Isomalt is 45 - 65 percent as sweet as sugar. Maltitol is 75 percent as sweet as sugar. Hydrogenated starch hydrolysates (HSH) are produced by the partial hydrolysis of corn. HSH are nutritive sweeteners that provide 40 - 90 percent of the sweetness of sugar.

In one embodiment of the invention, the sugar alcohol is selected from the group consisting of arabitol, erythritol, glycerol, mannitol, sorbitol, xylitol, lactitol, isomalt, maltitol, mannitol, and hydrogenated starch hydrosylates and mixtures thereof.

In one embodiment of the invention, the sugar alcohol is selected from maltitol or xylitol or mixtures thereof. The molar concentration of any of the sugar alcohols may be as defined above.

In the invention, the iron hydroxypyrone is pharmaceutically acceptable. This means that the iron hydroxypyrone is suitable for oral administration to a subject or patient in need of iron treatment.

In one embodiment, the iron hydroxypyrone is a neutral complex comprising iron cations and hydroxypyrone anions and without additional charge balancing anions, such as hydroxide or chloride. In an embodiment of the invention, the iron hydroxypyrone is an iron tri(hydroxypyrone) i.e., Fe(hydroxypyrone)₃, such as ferric tri(hydroxypyrone).

In an embodiment of the invention, the iron hydroxypyrone is a ferric tri(hydroxypyrone), where the hydroxypyrone is as defined herein, such as ferric trimaltol or ferric triethylmaltol.

By "neutral complex", it is intended to mean that the positive charge on the iron cation is balanced by the negative charge on the ligands in the complex. Therefore the total charge on the iron hydroxypyrone complex is zero. Because there is an internal balance of charges between the iron cation and the hydroxypyrone ligands, there is no need for any additional non-covalently bound anions, such as chloride, to balance any remaining charge on the iron cation.

In one embodiment of the invention, the iron hydroxypyrone compound comprises iron in the ferric (Fe³⁺) oxidation state.

When the iron is present in the ferric state, the neutral iron hydroxypyrone complex comprises hydroxypyrone and ferric iron in the stoichiometric ratio of 3:1 hydroxypyrone: ferric iron. The neutral complex of ferric iron and hydroxypyrone comprises three monobasic, bidentate hydroxypyrone ligands covalently bound to a ferric ion. The hydroxypyrone ligand is a bidentate ligand and is monobasic. The singly charged hydroxypyrone ligand contains an -O- group in place of the -OH group present in the neutral hydroxypyrone ligand.

The hydroxypyrone ligands in the iron hydroxypyrone may be the same or different. In a preferred embodiment, all of the hydroxypyrone ligands are the same.

Advantageously, the iron hydroxypyrone compound may or may not be completely or substantially free of charged ferric hydroxypyrone complexes and neutral mixed ligand ferric complexes comprising covalently bound carboxylate ligands.

By "charged ferric hydroxypyrone complexes", it is intended to mean ferric hydroxypyrone complexes in which the stoichiometric ratio of hydroxypyrone to ferric iron is 2:1 or 1:1 so that the charge on the ferric cation is not internally balanced by the charge on the hydroxypyrone ligand. The total charge on the complex may be +1 or +2 and at least one counterion, such as, for example, chloride will be required in order to balance the charge.

By "substantially free", it is meant that the charged ferric complexes or neutral mixed ligand ferric complexes comprising carboxylate ligands comprise less than 10 % by weight of the total weight of the iron species in the final composition, based on the composition, and preferably less than 5%, such as less than 2 wt.% or 1 wt.% or about 0 wt.%.

Where the iron hydroxypyrone compound has one or more chiral centres, the iron hydroxypyrone compound may be obtained as either pure enantiomer or diastereoisomer, a racemic mixture or a mixture enriched in either enantiomer or diastereoisomer. The mixture of enantiomers or diastereoisomers may be separated and purified using any of the known methods in the art. However, the mixture of optical isomers is typically not separated and purified.

In one embodiment, the hydroxypyrone in the present invention is a hydroxy-4-pyrone. For example, the hydroxy-4-pyrone can be a 3-hydroxy-4-pyrone or a 3-hydroxy-4-pyrone in which one or more of the hydrogen atoms attached to the ring carbon atoms is replaced by an aliphatic hydrocarbon group having 1 to 6 carbon atoms.

The substituted 3-hydroxy-4-pyrones may comprise more than one type of aliphatic hydrocarbon group. However, it is generally preferred if there is substitution by one rather than two or three aliphatic hydrocarbon groups.

In one embodiment, the hydroxypyrone ligand may be a 5-hydroxypyrone, such as Kojic acid (5-hydroxy-2-(hydroxymethyl)-4-pyrone). In a further embodiment, the hydroxypyrone used in the present invention may comprise mixtures of the hydroxypyrone ligands mentioned above.

In one embodiment of the invention, the hydroxypyrone does not comprise a hydroxymethyl, hydroxyethyl or hydroxyalkyl substituent, where the alkyl is preferably C1 to C10, such as C1 to C6. In one embodiment of the invention, the hydroxypyrone does not comprise or consist of Kojic acid.

The term "aliphatic hydrocarbon group" is used herein to include both acyclic and cyclic groups that may be unsaturated or saturated, the acyclic groups having a branched chain or preferably a straight chain. Particularly preferred groups are those having from 1 to 4 carbon atoms, more preferably those having from 1 to 3 carbon atoms. Saturated aliphatic hydrocarbon groups are preferred, these being either cyclic groups such as the cycloalkyl groups cyclopropyl, and particularly cyclohexyl, or more preferably acyclic groups such as methyl, ethyl, n-propyl and isopropyl. Methyl and ethyl are particularly preferred.

Substitution at the 2- or 6- position is of particular interest, although, when the ring is substituted by the larger aliphatic hydrocarbon groups, there may be an advantage in avoiding substitution on a carbon atom alpha to the system. This system is involved in the formation of a complex with iron and the close proximity of one of the larger aliphatic hydrocarbons may lead to steric effects that inhibit complex formation.

Preferred hydroxypyrone ligands present in complexes according to the present invention have the formula (I), specific hydroxypyrones of particular interest have the formulae (II) and (III): 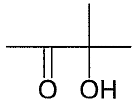 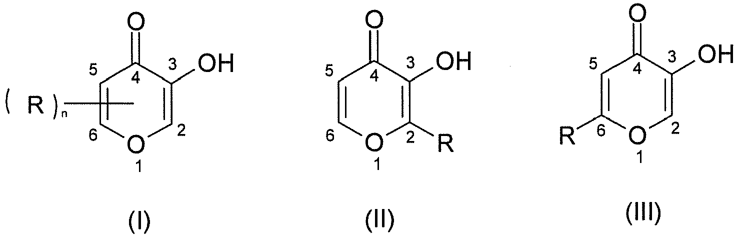 in which R is a cycloalkyl or alkyl group, for example, methyl, ethyl, n-propyl, isopropyl or butyl and n is 0, 1, 2 or 3 (the ring being unsubstituted by an alkyl group when n is 0).

Among these compounds, 3-hydroxy-2-methyl-4-pyrone (maltol; II, R = Me) is of most interest, whilst 3-hydroxy-4-pyrone (pyromeconic acid; I, n = 0), 3-hydroxy-6-methyl-4-pyrone (isomaltol, III, R = Me) and particularly 2-ethyl-3-hydroxy-4-pyrone (ethylmaltol; II, R = Et) are also of especial interest. For convenience, the compound 3-hydroxy-2-methyl-4-pyrone is referred to herein as "maltol".

In one embodiment of the invention the hydroxy-4-pyrone is selected from maltol, ethyl maltol or mixtures thereof. Maltol is most preferred and the iron hydroxypyrone compound used in the composition is preferably ferric trimaltol.

Certain hydroxypyrones, such as maltol, are available commercially. With others, a convenient starting material in many instances consists of 3-hydroxy-4-pyrone, which is readily obtainable by the decarboxylation of 2,6-dicarboxy-3-hydroxy-4-pyrone (meconic acid). For example, 3-hydroxy-4-pyrone may be reacted with an aldehyde to insert a 1-hydroxyalkyl group at the 2-position, which group may then be reduced to produce a 2-allyl-3-hydroxy-4-pyrone. Other preparative methods are described by Spielman, Freifelder, J. Am. Chem. Soc. Vol. 69, Page 2908 (1947).

The skilled person will appreciate that these are not the only routes to these hydroxypyrone compounds and that various alternatives known in the art may equally be used.

In an embodiment of the invention, the hydroxypyrone is a hydroxy-4-pyrone preferably selected from the group consisting of: a 3-hydroxy-4-pyrone and a 3-hydroxy-4-pyrone in which one or more of the hydrogen atoms attached to the ring carbon atoms are replaced by an aliphatic hydrocarbon group having 1 to 6 carbon atoms.

In one embodiment of invention, the hydroxypyrone is selected from maltol, ethyl maltol, or mixtures thereof.

In an embodiment of the invention, the composition further comprises a hydroxypyrone in addition to the iron hydroxypyrone. The hydroxypyrone may be as defined in any of the above embodiments but is preferably selected from maltol, ethyl maltol, or mixtures thereof.

In one embodiment of the invention, the pH of the composition is controlled to, for example, stabilize the iron hydroxypyrone. For example, the composition may include an acidity regulator, such as a buffer. For example, for ferric trimaltol the pH of the composition may advantageously be about 6 to about 8, for example from about 6.4 to about 7.4. For ferrous gluconate or ferrous fumarate and maltol, the pH of the composition may be less than about 6.5 to about 4 or 5, for example from about 5.8 to about 6.2.

In one embodiment of the invention, where the composition comprises an acidic flavouring agent, such as for example, apple or blackcurrant, the composition further includes an acidity regulator, such as a buffer as defined herein.

In an embodiment of the invention, the pH of the composition is greater than 4, 5 or 6. In one embodiment of the invention, the pH of the composition is from about 5 to about 8, such as from about 6 to about 7. In a particular embodiment, the pH of the composition is about 6, which is about neutral, or from about 5.5 to about 7.5. It is preferred that the pH of the composition is not strongly acidic.

In one embodiment of the invention, the composition comprises further hydroxypyrone. The further hydroxypyrone may be as defined herein and is preferably maltol or ethylmaltol. The further hydroxypyrone is added, typically in an uncomplexed form, to the composition in addition to the hydroxypyrone present in the iron hydroxypyrone. The further hydroxypyrone may be the same as or different from the hydroxypyrone in the iron hydroxypyrone. Typically, they are the same. The molar ratio of the further hydroxypyrone to the iron hydroxypyrone may be from about 10:1 to 1:10, such as about 5:1 to 1:5, 3:1 to 1:3, or 2:1 to 1:2.

In an embodiment of the invention, where the pH of the composition of the invention is less than about 7, such as when buffered by citric acid/Na Citrate, then the addition of further hydroxypyrone, such as maltol, will reduce disproportionation and ensure that the iron hydroxypyrone in the composition is mainly a 1:3 chelate.

In order to control the pH of the composition, for example in the above ranges, the composition may comprise a buffer or a pH-adjusting agent. In one embodiment, the buffering agent is selected from bicarbonates, sodium acetate, amino acids, such as lysine, and non-chelating weak organic acids. Typically the buffer is a salt prepared from an organic acid or base. Suitable examples of buffers include, for example, organic acid salts, such as salts of citric acid, ascorbic acid, gluconic acid, carbonic acid, tartaric acid, succinic acid, acetic acid, of phthalic acid; PIPES; or phosphate buffers. In one embodiment of the invention, the buffer system used is a citric acid/sodium citrate buffer in order to provide a pH of, for example, about 6. A citric acid/sodium phosphate buffer may also be used to provide a pH in the range of from about 6.4 to about 7.4.

In an embodiment of the invention, the weight ratio of the organic acid, such as citric acid, to the salt, such as citrate or phosphate in the composition may be in the range of from about 1:1 to about 1:15, such as from 1:2 to about 1:12. A buffer solution may be pre-prepared and added to the composition with such a weight ratio of components. The amount of a buffer solution added to the composition may be from about 0.1 to about 15 % (v/v), or from about 0.2 to about 10 % (v/v) or from about 1 to about 5 % (v/v) based on the total volume of the composition.

In one embodiment of the invention, the composition is in the form of a liquid or liquid suspension, comprising an iron hydroxypyrone, a taste masking agent and water, wherein the iron hydroxypyrone is present in the liquid or liquid suspension in a molar concentration of from about 0.05 to about 0.6 M, or from about 0.1 to about 0.5 M, or from about 0.12 to about 0.45 M, wherein the iron hydroxypyrone comprises or consists of a ferric trihydroxypyrone, such as ferric trimaltol, or a mixture of a ferrous carboxylate salt and a hydroxypyrone, such as maltol, and the taste masking agent comprises a sugar alcohol, or a non-saccharide sweetener such as stevia or aspartame, wherein the weight ratio, such as dry weight, of the iron hydroxypyrone to the sugar alcohol or non-saccharide sweetener is preferably from about 1 to about 20, and wherein the pH of the composition is preferably from about 5 to about 7.

The composition of the invention is typically a pharmaceutical composition. By the term "pharmaceutical composition", it is intended to mean a composition which is suitable for administration to a subject or patient, such as in need of iron treatment. By the term "subject" or "patient" we include an animal, such as a mammal, for example a human. Other examples of mammals include cats, dogs, sheep, cows, horses and monkeys. The terms "subject" and "patient" may refer to the same animal.

In one embodiment of the invention, the composition of the invention, as described in any of the embodiments disclosed herein, comprises a sweetness enhancer. One example of a suitable sweetness enhancer is a hydroxypyrone as described herein. This may be added separately from the iron hydroxypyrone. The sweetness enhancer preferably comprises maltol or ethyl maltol, or combinations thereof. In one embodiment of the invention, the sweetness enhancer is present in the composition in an amount of from about 0.1 to about 15 % (w/v), or from about 0.2 to about 10 % (w/v) or from about 1 to about 5 % (w/v) based on the total volume of the composition.

In an embodiment of the invention, when the composition is or comprises a liquid suspension, the composition further comprises a suspending agent. A suspending agent helps to reduce the sedimentation rate of particles in suspension. These are insoluble particles that are dispersed in a liquid medium. Suitable examples of suspending agents include methylcellulose, carboxy methyl cellulose, sodium alginate or povidone and combinations thereof. The amount of the suspending agent may be from about 0.01 to about 15 % (w/v), or from about 0.02 to about 10 % (w/v) or from about 0.1 to about 5 % (w/v) based on the total volume of the composition.

In one embodiment of the invention, the composition further comprises a flavouring agent. The flavour may be any suitable flavour. The amount of the flavouring agent may be from about 0.01 to about 15 % (w/v), or from about 0.02 to about 10 % (w/v) or from about 0.1 to about 5 % (w/v) based on the total volume of the composition. The flavour may, for example, be selected from the group consisting of apple, blackcurrant, orange, lemon, grape, maple, raspberry, cherry, menthol, peppermint, spearmint, vanilla, chocolate, and strawberry and combinations thereof. The flavouring agent may be any commercially available flavouring agent. In one embodiment of the invention, the taste masking and flavouring agent comprises an apple and blackcurrant mixture, such as from a fruit concentrate, optionally with a non-saccharide sweetener such as stevia. This may be used in combination with an iron hydroxypyrone comprising a ferrous salt and a hydroxypyrone or an iron hydroxypyrone comprising a complex of iron and a hydroxypyrone, as defined herein.

The composition of the invention is generally coloured. The typical colour of the composition is red although it may also be brownish, purple or burgundy.

The composition may also include a colour masking agent. Colour masking agents may conceal the colour of the composition without such an agent. Suitable examples of such agents include, for example, amaranth, blackcurrant, strawberry or raspberry colourings. These colourings may conceal the red or purple colour of the composition. The amount of the colouring agent may be from about 0.01 to about 15 % (w/v), or from about 0.02 to about 10 % (w/v) or from about 0.1 to about 5 % (w/v) based on the total volume of the composition.

In one embodiment of the invention the composition comprises a liquid or liquid medium which is selected from water or an oil, or mixtures thereof. The water may be water from a public or private supply, such as a tap, and may be water which has been subjected to pharmaceutical purification. In general the liquid, such as water or an edible oil, is suitable for oral administration. The oil may be any edible oil. The liquid is preferably sterile and pyrogen-free: examples are saline and water. The composition of the invention may be particularly suitable for oral and parenteral administration. Typically, the composition comprises water as the liquid component of the composition.

In one embodiment of the invention the composition is for oral administration and not for parenteral administration, such as injection or infusion. In one embodiment of the invention the liquid composition according to the invention is suitable for delivery via a nasopharyngeal or gastric feeding tube.

In an embodiment of the invention, the composition is for oral administration to an animal such as a mammal, for example a human. In one embodiment of the invention, the animal, such as a mammal has a renal disorder or is diabetic. The composition may be for administration to children (for example, under the age of 12, such as from 5 to 11 years old) and is preferably packaged and labelled for children. The composition may also be suitable for administration to adults who cannot swallow tablets, or have swallowing difficulties. This swallowing difficulty may be for neurological reasons such as, for example, a stroke, multiple sclerosis (MS); or for subjects who have had oesophageal or throat surgery or radiation or who have inflamed mucosa such as mucositis.

The composition of the invention may also comprise a sweetening agent. This may be the same as or different from the taste masking agent. The sweetening agent may be a saccharide or non-saccharide sweetening agent. Suitable sweeteners include, for example, one or more of aspartame, stevia-based sweetener extract, saccharin, refined sugar, neohesperidine dihydrochalcone and hesperidine dihydrochalcone 4'-β-D glucoside, or mixtures thereof. The amount of the sweetening agent may be as defined above for the taste masking agent.

In an embodiment of the invention, the composition further comprises a sweetening agent and a colour masking agent, such as a blackcurrant and/or raspberry colouring.

The composition of the invention may be for administration to subjects with chronic kidney disease (CKD), subjects with diabetes, such as Type I or Type II diabetes or subjects who are children (for example, under the age of 12, such as from 5 to 11 years old). The subject may be an animal as defined herein and is preferably a mammal such as a human.

In one embodiment of the invention, the subject has or is at risk of developing anaemia, such as iron deficiency anaemia or Vitamin B12 anaemia. The anaemia may be associated with blood loss, such as following surgery, or an inflammatory disease of the gastrointestinal tract, or anaemia associated with pregnancy or a poor diet.

The composition of the invention is especially useful for oral administration to patients who have difficulty in swallowing solid forms. Such difficulties are common in patient groups such as children and geriatrics.

In one embodiment, the method of forming a liquid or liquid suspension, such as according to the invention, comprises combining an iron hydroxypyrone, preferably in a solid or dry form, such as a powder, with a liquid and a taste masking agent. The method can comprise, for example, combining the iron hydroxypyrone with a taste masking agent to form a dry mixture, and then combining the dry mixture with a liquid such as water or oil. The method of the invention preferably does not comprise any drying step. The terms "dry" or "dried" may refer to compositions which comprise less than about 15 wt.% liquid, such as water, or less than about 10 wt.%, or less than 5 wt.%, or less than about 1 wt.%, such as about 0.5 wt.% or about 0 wt.% liquid, based on the total weight of the composition.

The dry, dried or powder components of the formulation may be combined with the liquid components of the formulation at the point of dispensing, such as in a pharmacy, delivery or consumption. For example, the liquid may be part of a kit of parts, separate from the dry, dried or powder components or could be provided, separately from the dry, dried or powder components, by a dispenser, such as a pharmacist, or consumer. For example, a consumer or patient may add water to the dry, dried or powder components and make up the liquid or liquid suspension formulations according to the invention. The liquid components may be sold separately from or together with the dried components.

The dry, dried or powder components of the formulation may comprise the iron hydroxypyrone alone or in combination with one or more of the taste masking agent, flavouring agent, colouring agent, suspending agent, or combinations thereof.

The liquid components of the formulation may comprise the liquid and one or more of the taste masking agent, flavouring agent, colouring agent, suspending agent, or combinations thereof.

In one embodiment of the invention, the liquid and the taste masking agent may be comprised in the same solution, as for example a concentrate. For example, the iron hydroxypyrone may be combined with a concentrate, such as a fruit or vegetable concentrate, to form a liquid or liquid suspension. The liquid or liquid suspension may then be diluted with the same or a different liquid from in the concentrate to provide a suitable concentration of the components. Typically, the combination of an iron hydroxypyrone and a concentrate will be diluted with water. Suitable examples of concentrate include, for example, fruit concentrate, such as apple, orange, pineapple, or blackcurrant concentrate.

The method may additionally comprise the step of diluting the composition formed by combining the components. The dilution factor (molar concentration after dilution/molar concentration before dilution) may be, for example, less than about 0.1, 0.2, 0.5, or 0.01. In one embodiment of the invention there may be no dilution of the composition. The dilution is preferably carried out using water or a liquid comprising water.

The dilution may increase the solubility of the iron hydroxypyrone in the liquid, such as water, if this is desired and provide a clear solution. This could also be achieved by decreasing the pH of the composition, such as to less than about 7, or less than about 6.

Alternatively, the iron hydroxypyrone and the taste masking agent may be combined separately with the liquid, such as water or an oil. The iron hydroxypyrone may be combined with the liquid before or after the taste masking agent. The remaining components of the composition may be added before or after the iron hydroxypyrone and the taste masking agent. If the composition is a liquid suspension, then a suspending agent, such as defined above, may preferably be added before the iron hydroxypyrone.

Where the concentration of the iron hydroxypyrone is close to or exceeds the solubility in a particular liquid, such as water, at about room temperature, such as 5 to 40°C, the pH of the liquid may be lowered to less than about 7, 6 or 5 and a hydroxypyrone may be added. The hydroxypyrone may the same or different from the hydroxypyrone of the iron hydroxypyrone but typically it is the same. The amount of the hydroxypyrone may be an equimolar amount or greater based on the amount of iron hydroxypyrone.

More than one iron hydroxypyrone compound may be contained in the composition, such as a pharmaceutical composition, and other active compounds may also be included. Typical additives include compounds having the ability to facilitate the treatment of anaemia, such as folic acid. A zinc source may also be included. The iron hydroxypyrone may be the only pharmaceutically active component present in the composition. For example, the iron hydroxypyrone may be the only source of iron present in the composition.

Preferably the above compositions, as defined in any of the above embodiments, are suitable for use in medicine.

In one embodiment of the invention, the composition is in the form of a liquid or liquid suspension or semisolid food product. For example, the composition of the invention may be in the form of a beverage, or a foodstuff, such as a yoghurt.

Whilst the dosage of the composition given in each particular case will depend upon various factors, including the particular components of the composition, it may be stated by way of guidance that maintenance at a satisfactory level of the amount of iron present in the human (or animal) body will often be achieved using a daily dosage, in terms of the iron content of the compound, which lies in a range from about 1 to 150 mg, such as from 10 to 120 mg (preferably as iron). However, it may be appropriate in certain cases to give daily dosages either below or above these levels. Compositions containing 15 to 50 mg iron, to be taken once daily, twice daily or three times daily (depending on the severity of the anaemia) are, for example, suitable for the treatment of anaemia. The amount of the composition of the invention which represents a unit dose may be, for example, from about 5 ml to about 10 ml, such as about 5 ml or about 10 ml. Each unit dose may comprise, for example, from about 10 to about 120 mg of iron, such as for example, from about 20 mg to about 80 mg iron. The unit dose may be from, for example, 90 to 120 mg of iron.

In one embodiment of the invention, the composition provides a dose of iron of from about 10 mg to about 120 mg, such as from 60 to 90 mg, in from about 5 ml to about 10 ml of the composition.

The composition of the invention may be packaged and/or labelled. In one embodiment of the invention, the composition may be packaged in the form of: a bottle, which could be, for example, glass or plastic; a sachet; or a carton, such as made from a plastic or plastic coated paper; or cardboard.

The packaging may optionally comprise instructions for a patient to dilute the composition before administration or this may form part of the use of the composition or method of administration. The dilution factor (molar concentration after dilution/molar concentration before dilution) may be, for example, less than about 0.1, 0.2, 0.5, or 0.01. In one embodiment of the invention there may be no dilution of the composition. The dilution is preferably carried out using water or a liquid comprising water.

The compositions of the invention suitably contain from 0.1% to 20% by weight iron, such as 0.1% to 10% by weight, for example, preferably 2 to 10% by weight.

The compositions of the present invention are particularly useful for mild and serious anaemias. Many of the patients with such disorders are intolerant of standard ferrous anti-anaemia compounds. Ferrous preparations are contra-indicated or the subject of warnings in such conditions. Furthermore, patients who may need blood transfusions or in-patient treatment with intravenous injections can be treated on an outpatient basis saving substantial costs of treatment.

In addition, the compositions of the invention may be for the treatment of anaemia in children with, for example, Stevens-Johnson syndrome.

The compositions of the invention may be used in a method for the treatment of a subject to effect an increase in the levels of iron in the subject's body, such as the bloodstream, and/or the prevention and/or treatment of anaemia, such as iron-deficiency anaemia, which comprises administering to said subject an effective amount of the composition as defined previously.

In one embodiment, the compositions of the invention may be administered to subjects or patients with a gastric pH greater than 4. Such patients are disclosed in WO 2009/138761,

The following non-limiting examples illustrate the invention and do not limit its scope in any way. In the examples and throughout this specification, all percentages, parts and ratios are by weight unless indicated otherwise. Average molecular weights are based on weight unless otherwise specified. It will be appreciated that the various percentage amounts of the different components that are present in the products of the invention, including any optional components, will add up to 100%.

### EXAMPLES:

### Example 1

### ACCEPTABILITY STUDY: Ferric Trimaltol solution

### INTRODUCTION

Taste and smell are usually classified together as the "chemical senses". Taste is also linked with smell because of the close psychological relation of the two senses as evidenced by the frequent subjective confusion between olfactory and gustatory sensations. Many chemical stimuli also affect the end organs of touch, pressure, temperature and pain receptors in those organs. The astringent sensation derived from many acids is usually said to be a purely tactual sensation.

Pharmaceutical products are often formulated to overcome disadvantages of taste once this has been recognised. Bitter, sour, metallic and musty tastes are particularly problematical and in some cases outright irritancy needs to be masked. Some common iron products are notorious for their "metallic" sour/bitter taste. Thus, most ferrous solid dose preparations are enteric coated or film coated or marketed as capsules because of the problem. Liquid preparations such as ferrous sulphate elixir (USP 1995) contain sucrose and ferrous gluconate mixture (Martindale 1989) contains glucose. The mixtures also need to be acidic to maintain stability of the iron compound. Poor acceptability is known to affect patient compliance and liquid preparations are often administered to children and individuals in poor health. The study therefore investigated the problem by application of solutions to the tongue in regions where there are selective receptors taste features.

### Taste

The zonal distribution of sensitivity complicates the thresholds for various modalities because the threshold varies with the region of the tongue being tested. Calculations were made which suggested that the concentration of iron preparations to be tested should be 10⁻³ M and 10⁻⁴ M since these were the likely concentrations arising after oral dosing as iron in the product form.

The following solutions were used, made up in tap water.

**Solution Number and Concentration**

| | | |
|---|---|---|
| Sugar | [1] 10⁻² M | [2] 10⁻³ M |
| FeS04 | [3] 10⁻³ M | [4] 10⁻⁴ M |
| Fe[Maltol]₃ | [5] 10⁻³ M | [6] 10⁻⁴ M |
| Tap water | [7] | [8] |

Solution was applied to the tongue with a soaked cotton wool bud and the subject (blindfolded) with the mouth open and tongue outstretched was asked to indicate the letters:
(a) - sweet (b) - salty (c) - sour/metallic (d) - bitter and metallic (e) - no taste
depending on which quality had been perceived. Each area of the tongue was tested several times with each concentration, after each set the mouth was rinsed out with tap water.

| Area | Subject | Solution | 1 | 2 | 3 | 4 | 5 | 6 | 7 | 8 |
|---|---|---|---|---|---|---|---|---|---|---|
| Tip | (i) | | a | a | c | c | e | e | e | e |
| | (ii) | | a | a | d | d | e | e | e | e |
| | (iii) | | a | e | c | c | c | e | e | e |
| | (iv) | | a | e | e | c | c | d | e | e |
| Base | (i) | | a | a | c | e | e | e | e | e |
| | (ii) | | a | e | c | c | c | e | e | e |
| | (iii) | | a | a | d | e | e | e | e | e |
| | (iv) | | a | e | c | e | d | d | e | e |
| Right half | (i) | | a | a | c | e | d | e | e | e |
| | (ii) | | a | a | c | d | e | e | e | e |
| | (iii) | | a | a | d | d | d | e | e | e |
| | (iv) | | a | e | c | d | d | e | e | e |

### Results

| Sugar | | Fe S04 | | Fe tri-maltol | | Water | |
|---|---|---|---|---|---|---|---|
| 1 | 2 | 3 | 4 | 5 | 6 | 7 | 8 |
| 12a | 7a | 8c | 4c | 3c | 2d | 12e | 12e |
| | 5e | 3d | 4d | 4d | 10e | | |
| | | 1e | 4e | 5e | | | |
| 19a 5e | | 12c 7d 5e | | 3c 6d 15e | | 24e | |

### Conclusion

All four subjects clearly distinguished the sweet solutions and recognised the sour (metallic) taste of iron sulphate. Iron tri-maltol was indistinguishable from tap water in 15 tests and recognized as slightly metallic in 9 tests.

As a result of this test two volunteers took a 10ml dose of a liquid solution of ferric trimaltol containing 30mg as iron. At this dose the solution had a slightly metallic/caramel taste.

Short term (5 mins) exposure of iron sulphate or iron tri-maltol on grazed skin in two volunteers at concentrations of 10⁻⁴ M did not induce irritation. Similarly, neither iron tri-maltol or iron sulphate in contact with the nasal mucosa for 10 minute periods proved to be irritant though the metallic sensation of the sulphate was perceived. In a separate taste study using solid preparations of iron tri-maltol a faint but lingering caramel/ sweet taste was noted.

### Example 2:

A liquid formulation was developed for administration to children with Stevens Johnson syndrome. This was a ferrous fumarate/ maltol composition. This formulation also included blackcurrant juice since the iron preparation itself is red and thus any disproportionation is masked. The results were most encouraging since these children cannot take any solid dosage forms. They accepted the formulation and showed a positive response to treatment.

### Example 3:

An example of a suitable composition according to the invention is as follows:

| | |
|---|---|
| FeM | 3.5 g |
| Maltitol | 25 g |
| Suspending Agent | qv |
| Raspberry Flavour | qv |
| Buffer | qv |
| Water | 100 ml |

### Example 4:

Method of synthesis of iron hydroxypyrones are described in US 6 635,631 B2 and US 7, 135,196 B2.

A liquid iron product for administration as a pharmaceutical entity requires a stable and acceptable and palatable formulation which can be administered to a broad patient population. Current iron products of ferrous sulphate (pH 1.8-5.3) and ferrous gluconate in liquid form (pH 3.4-3.8) have a very low pH (USP 1995) for stability of the iron compound and a very high concentration of sucrose is required to mask the bitter metallic taste. These formulations do not have a good acceptability and clinical trials report a 25% drop out rate due to intolerance of the product form.

Unlike the ferrous products a maximum stability of the liquid ferric pyrones and ferrous products containing maltol is achieved in the pH range 5.5 - 8. With this knowledge the formulations developed require ingredients suited to this constraint and also standardisation of colour and taste for patient acceptability. At higher concentrations of active a suspension formulation may be preferred. Furthermore the excipients must not reduce the excellent bio-availability of iron from iron pyrone formulations.

The following examples represent examples of formulations which meet the criteria as set out above:

| The product volume has been standardised at 100ml with a dosage volume at 5ml. | | |
|---|---|---|
| Ferrous fumarate | 1.1g | (equivalent to 350mg as iron) |
| Maltol sweetness enhancer | 1.0g-2.5g | |
| Stevia sweetening agent | 5g-20g | |
| Citric acid/sodium citrate qv | 1/5 -1/12 ratio | pH 5.8-6.2 |
| Carboxy methyl cellulose | | |
| Colouring agent amaranth qv | | |
| Water | To 100ml | 35mg of iron in a 10 ml dose |

| The product volume has been standardised at 100ml with a dosage volume at 5-10ml. | | |
|---|---|---|
| Ferrous gluconate | 2.6g | (equivalent to 300 mg as iron) |
| Maltol sweetness enhancer | 1.0g- 5.0g | |
| maltitol sweetening agent | 5g-40g | |
| Citric acid/sodium citrate qv | 1/5 -1/12 ratio | pH 5.8-6.2 |
| Sodium alginate | 500mg-2g | |
| Colouring agent amaranth qv | | |
| Blackcurrent juice concentrate qv | 10ml | |
| Water | To 100ml | 15mg of iron in a 5 ml dose |

| The product volume has been standardised at 100ml with a dosage volume at 5-10ml. | | |
|---|---|---|
| Ferric trimaltol | 4.6g | (equivalent to 600mg as iron) |
| Maltol sweetness enhancer | 0.2g-2.0g | |
| xylitol sweetening agent | 10g-60g | |
| Citric acid/sodium phosphate qv | 1/2 -1/10 ratio | pH 6.4-7.4 |
| Carboxy methyl cellulose | | |
| Colouring agent amaranth qv | | |
| Peppermint flavouring agent qv | 0.2-1.0ml | |
| Water | To 100ml | 30 mg of iron in a 5 ml dose |

## Claims

1. A pharmaceutical composition in the form of a liquid or liquid suspension for oral administration, comprising an iron hydroxypyrone and a taste masking agent, wherein the iron hydroxypyrone is present in the liquid or liquid suspension in a concentration of at least about 10⁻⁵ moles per litre, and wherein the taste masking agent is present in an amount effective to mask the metallic taste associated with the iron hydroxypyrone.

2. The composition according to Claim 1, wherein the taste masking agent does not comprise a sugar and/or wherein the iron hydroxypyrone is present in the liquid or suspension in a molar concentration of at least about 10⁻² M to about 10⁻¹ M.

3. The composition according to Claim 1 or Claim 2, wherein the taste masking agent comprises a sugar alcohol, stevia, a non-saccharide sweetener, such as aspartame, or combinations thereof.

4. The composition according to Claim 3, wherein the sugar alcohol is selected from the group consisting of arabitol, erythritol, glycerol, mannitol, sorbitol, xylitol, lactitol, isomalt, maltitol, mannitol, and hydrogenated starch hydrosylates and mixtures thereof, preferably wherein the sugar alcohol is maltitol or xylitol or mixtures thereof.

5. The composition of any one of Claims 1 to 4, wherein the hydroxypyrone is a hydroxy-4-pyrone, preferably wherein the hydroxy-4-pyrone is selected from the group consisting of: a 3-hydroxy-4-pyrone and a 3-hydroxy-4-pyrone in which one or more of the hydrogen atoms attached to the ring carbon atoms are replaced by an aliphatic hydrocarbon group having 1 to 6 carbon atoms.

6. The composition according to any one of Claims 1 to 5, wherein the hydroxypyrone is selected from maltol, ethyl maltol, or mixtures thereof, and/or wherein the iron hydroxypyrone compound comprises an iron tri(hydroxypyrone), such as ferric tri(hydroxypyrone), for example ferric trimaltol, or the iron hydroxypyrone comprises a ferric or a ferrous salt and a hydroxypyrone.

7. The composition according to any one of Claims 1 to 6, wherein the pH of the composition is from about 5 to about 8.

8. The composition according to any one of Claims 1 to 7, wherein the taste masking agent is present in a molar concentration of at least about 0.1 M, such as about 0.5 M.

9. The composition according to any one of Claims 1 to 8, wherein the composition further comprises a suspending agent or wherein the composition comprises further hydroxypyrone, and/or wherein the composition further comprises a flavouring agent.

10. The composition according to any one of Claims 1 to 9, wherein the composition comprises a liquid selected from water, an oil, or mixtures thereof.

11. The composition according to any one of Claims 1 to 10, wherein the composition is for oral administration to a mammal, such as a human and/or wherein the composition does not comprise ascorbic acid or derivatives thereof.

12. A composition according to any one of Claims 1 to 11, for use in medicine.

13. A composition according to any one of Claims 1 to 11, for use in treating and/or preventing anaemia, or for increasing the level of iron in a subject's body.

14. The composition for use according to Claim 13, wherein the anaemia is iron deficiency anaemia.

15. A kit of parts comprising: an iron hydroxypyrone in the form of a solid; one or more liquids; and one or more taste masking agents, wherein the amount of the iron hydroxypyrone and the one or more liquids is such that, when combined, a liquid or liquid suspension is formed in which the iron hydroxypyrone is present in a concentration of at least about 10⁻⁵ moles per litre, and wherein the taste masking agent is present in an amount effective to mask the metallic taste associated with the iron hydroxypyrone.

## Patentansprüche

1. Pharmazeutische Zusammensetzung in der Form einer Flüssigkeit oder einer flüssigen Suspension zum oralen Verabreichen, umfassend ein Eisenhydroxypyron und ein Geschmacksmaskierungsmittel, wobei das Eisenhydroxypyron in der Flüssigkeit oder der flüssigen Suspension in einer Konzentration von mindestens etwa 10⁻⁵ Mol pro Liter vorliegt, und wobei das Geschmacksmaskierungsmittel in einer Menge vorliegt, die wirksam ist, um den mit dem Eisenhydroxypyron verbundenen metallischen Geschmack zu maskieren.

2. Zusammensetzung nach Anspruch 1, wobei das Geschmacksmaskierungsmittel keinen Zucker umfasst und/oder wobei das Eisenhydroxypyron in der Flüssigkeit oder der Suspension in einer Stoffmengenkonzentration von mindestens etwa 10⁻² mol/l bis etwa 10⁻¹ mol/l vorliegt.

3. Zusammensetzung nach Anspruch 1 oder Anspruch 2, wobei das Geschmacksmaskierungsmittel einen Zuckeralkohol, Stevia, einen Nicht-Saccharid-Süßstoff wie Aspartam oder Kombinationen davon umfasst.

4. Zusammensetzung nach Anspruch 3, wobei der Zuckeralkohol aus der Gruppe bestehend aus Arabit, Erythrit, Glycerin, Mannit, Sorbit, Xylit, Lactit, Isomalt, Maltit, Mannit und hydrierten Stärkehydrosylaten und Gemischen davon ausgewählt ist, wobei der Zuckeralkohol vorzugsweise Maltit oder Xylit oder Gemische davon ist.

5. Zusammensetzung nach einem der Ansprüche 1 bis 4, wobei das Hydroxypyron ein Hydroxy-4-pyron ist, wobei das Hydroxy-4-pyron vorzugsweise aus der Gruppe ausgewählt ist, die aus Folgendem besteht:
einem 3-Hydroxy-4-pyron und einem 3-Hydroxy-4-pyron, bei dem eines oder mehrere der an die Ringkohlenstoffatome gebundenen Wasserstoffatome durch eine aliphatische Kohlenwasserstoffgruppe mit 1 bis 6 Kohlenstoffatomen ersetzt sind.

6. Zusammensetzung nach einem der Ansprüche 1 bis 5, wobei das Hydroxypyron aus Maltol, Ethylmaltol oder Gemischen davon ausgewählt ist und/oder wobei die Eisenhydroxypyronverbindung ein Eisen-tri(hydroxypyron) wie Eisen(III)-tri(hydroxypyron), beispielsweise Eisen(III)-tri(maltol), umfasst oder das Eisenhydroxypyron ein Eisen(III)-Salz oder ein Eisen(II)-Salz und ein Hydroxypyron umfasst.

7. Zusammensetzung nach einem der Ansprüche 1 bis 6, wobei der pH-Wert der Zusammensetzung etwa 5 bis etwa 8 beträgt.

8. Zusammensetzung nach einem der Ansprüche 1 bis 7, wobei das Geschmacksmaskierungsmittel in einer Stoffmengenkonzentration von mindestens etwa 0,1 mol/l, wie etwa 0,5 mol/l, vorliegt.

9. Zusammensetzung nach einem der Ansprüche 1 bis 8, wobei die Zusammensetzung ferner ein Suspendierungsmittel umfasst oder wobei die Zusammensetzung weiteres Hydroxypyron umfasst und/oder wobei die Zusammensetzung ferner einen Aromastoff umfasst.

10. Zusammensetzung nach einem der Ansprüche 1 bis 9, wobei die Zusammensetzung eine Flüssigkeit umfasst, die aus Wasser, einem Öl oder Gemischen davon ausgewählt ist.

11. Zusammensetzung nach einem der Ansprüche 1 bis 10, wobei die Zusammensetzung zum oralen Verabreichen an ein Säugetier wie einen Menschen bestimmt ist und/oder wobei die Zusammensetzung keine Ascorbinsäure oder Derivate davon umfasst.

12. Zusammensetzung nach einem der Ansprüche 1 bis 11 zur Verwendung in der Medizin.

13. Zusammensetzung nach einem der Ansprüche 1 bis 11 zur Verwendung bei einem Behandeln und/oder einem Vorbeugen von Anämie oder zu einem Erhöhen des Eisengehalts im Körper eines Subjekts.

14. Zusammensetzung zur Verwendung nach Anspruch 13, wobei die Anämie eine Eisenmangelanämie ist.

15. Teilesatz, umfassend:
ein Eisenhydroxypyron in der Form eines Feststoffs;
eine oder mehrere Flüssigkeiten; und
ein oder mehrere Geschmacksmaskierungsmittel, wobei die Menge des Eisenhydroxypyrons und der einen oder mehreren Flüssigkeiten derart ist, dass in Kombination eine Flüssigkeit oder eine flüssige Suspension gebildet wird, in der das Eisenhydroxypyron in einer Konzentration von mindestens etwa 10⁻⁵ Mol pro Liter vorliegt und wobei das Geschmacksmaskierungsmittel in einer Menge vorliegt, die wirksam ist, um den mit dem Eisenhydroxypyron verbundenen metallischen Geschmack zu maskieren.

## Revendications

1. Composition pharmaceutique sous la forme d'un liquide ou d'une suspension liquide pour administration orale, comprenant une hydroxypyrone de fer et un agent masquant le goût, dans laquelle l'hydroxypyrone de fer est présente dans le liquide ou la suspension liquide à une concentration d'au moins environ 10⁻⁵ moles par litre, et dans laquelle l'agent masquant le goût est présent en une quantité efficace pour masquer le goût métallique associé à l'hydroxypyrone de fer.

2. Composition selon la revendication 1, dans laquelle l'agent masquant le goût ne comprend pas de sucre et/ou dans laquelle l'hydroxypyrone de fer est présente dans le liquide ou la suspension à une concentration molaire comprise entre au moins environ 10⁻² M et environ 10⁻¹ M.

3. Composition selon la revendication 1 ou la revendication 2, dans laquelle l'agent masquant le goût comprend un alcool de sucre, la stévia, un édulcorant non saccharidique, tel que l'aspartame, ou leurs combinaisons.

4. Composition selon la revendication 3, dans laquelle l'alcool de sucre est choisi dans le groupe constitué par l'arabitol, l'érythritol, le glycérol, le mannitol, le sorbitol, le xylitol, le lactitol, l'isomalt, le maltitol, le mannitol et les hydrolysats d'amidon hydrogéné et leurs mélanges, de préférence dans laquelle l'alcool de sucre est le maltitol ou le xylitol ou leurs mélanges.

5. Composition selon l'une quelconque des revendications 1 à 4, dans laquelle l'hydroxypyrone est une hydroxy-4-pyrone, de préférence dans laquelle l'hydroxy-4-pyrone est choisie dans le groupe constitué par : une 3-hydroxy-4-pyrone et une 3-hydroxy-4-pyrone dans laquelle un ou plusieurs des atomes d'hydrogène liés aux atomes de carbone du cycle sont remplacés par un groupe hydrocarboné aliphatique ayant 1 à 6 atomes de carbone.

6. Composition selon l'une quelconque des revendications 1 à 5, dans laquelle l'hydroxypyrone est choisie parmi le maltol, l'éthyl maltol ou leurs mélanges, et/ou dans laquelle le composé d'hydroxypyrone de fer comprend une tri(hydroxypyrone) de fer, telle que la tri(hydroxypyrone) ferrique, par exemple le trimaltol ferrique, ou l'hydroxypyrone de fer comprend un sel ferrique ou ferreux et une hydroxypyrone.

7. Composition selon l'une quelconque des revendications 1 à 6, dans laquelle le pH de la composition est compris entre environ 5 et environ 8.

8. Composition selon l'une quelconque des revendications 1 à 7, dans laquelle l'agent masquant le goût est présent en une concentration molaire d'au moins environ 0,1 M, par exemple environ 0,5 M.

9. Composition selon l'une quelconque des revendications 1 à 8, dans laquelle la composition comprend en outre un agent de suspension ou dans laquelle la composition comprend en outre de l'hydroxypyrone, et/ou dans laquelle la composition comprend en outre un agent aromatisant.

10. Composition selon l'une quelconque des revendications 1 à 9, dans laquelle la composition comprend un liquide choisi parmi l'eau, une huile ou leurs mélanges.

11. Composition selon l'une quelconque des revendications 1 à 10, dans laquelle la composition est destinée à une administration orale à un mammifère, tel qu'un humain et/ou dans laquelle la composition ne comprend pas d'acide ascorbique ou ses dérivés.

12. Composition selon l'une quelconque des revendications 1 à 11, destinée à être utilisée en médecine.

13. Composition selon l'une quelconque des revendications 1 à 11, destinée à être utilisée dans le cadre du traitement et/ou de la prévention de l'anémie, ou pour augmenter le niveau de fer dans le corps d'un sujet.

14. Composition destinée à être utilisée selon la revendication 13, dans laquelle l'anémie est une anémie ferriprive.

15. Kit de constituants comprenant : une hydroxypyrone de fer sous la forme d'un solide ; un ou plusieurs liquides ; et un ou plusieurs agents masquant le goût, dans lequel la quantité d'hydroxypyrone de fer et du ou des liquides est telle que, lorsqu'ils sont combinés, un liquide ou une suspension liquide est formée dans laquelle l'hydroxypyrone de fer est présente à une concentration d'au moins environ 10⁻⁵ moles par litre, et dans lequel l'agent masquant le goût est présent en une quantité efficace pour masquer le goût métallique associé à l'hydroxypyrone de fer.
